# EUROPÄISCHE PATENTANMELDUNG

(11) **EP 3 900 567 A1**
(43) Veröffentlichungstag der Anmeldung: **27.10.2021**
(21) Anmeldenummer: 21169954.1
(22) Anmeldetag: 22.04.2021
(51) Int. Cl.: A43B 7/14, A43B 23/02, A61F 5/01

(54) **ORTHOPÄDISCHER SCHUH**

(30) Priorität: 22.04.2020 LU 101760
(71) Anmelder: Schmidt, Bernd, 65812 Bad Soden am Taunus (DE)
(72) Erfinder: Schmidt, Bernd, 65812 Bad Soden am Taunus (DE)
(74) Vertreter: Habermann Intellectual Property Partnerschaft von Patentanwälten mbB

(57) **Zusammenfassung**

Die Erfindung betrifft einen orthopädischen Schuh (1) zur Stabilisierung eines Fußes in einem oberen Fußbereich und/oder im Knöchelbereich eines Trägers. Der orthopädische Schuh (1) weist einen Schuhschaft (2) auf, der sich mindestens entlang der Seiten des Fußes und über den oberen Fußbereich erstreckt. Der Schuhschaft (2) weist mindestens zwei Luftkissen (6) auf. Das eine Kissen ist medial, das heißt mittig zur Körperlinie und das andere Kissen lateral, das heißt seitlich zur Körperlinie angeordnet. Beide Luftkissen (6) sind durch eine Luftleitung (8) miteinander verbindbar. Mindestens ein Luftkissen (6) weist eine Luftzuführleitung (9) und ein Rückschlagventil (10) zum Verbinden mit einer Pumpeinrichtung (11) auf. Das Luftkissen (6) weist zwei Seitenteile (16, 17) auf, die eine Luftkammer ausbilden. Das innere Seitenteil (16) ist elastisch und das äußere Seitenteil (17) ist unelastisch ausgeführt.

## Beschreibung

Die Erfindung betrifft einen orthopädischen Schuh zur Stabilisierung eines Fußes in einem oberen Fußbereich und/oder im Knöchelbereich eines Trägers, wobei der orthopädische Schuh einen Schuhschaft aufweist, wobei sich der Schuhschaft mindestens entlang der Seiten des Fußes und über den oberen Fußbereich erstreckt, wobei der Schuhschaft mindestens zwei Luftkissen aufweist, wobei das eine Kissen medial, das heißt mittig zur Körperlinie und das andere Kissen lateral, das heißt seitlich zur Körperlinie angeordnet ist, wobei beide Luftkissen durch eine Luftleitung miteinander verbindbar sind, wobei mindestens ein Luftkissen eine Luftzuführleitung und ein Rückschlagventil zum Verbinden mit einer Pumpeinrichtung aufweist.

Patienten können an durch unterschiedliche Erkrankungen ausgelöste Fußformänderungen im Fußbereich oder an dauerhaft schwellendem Gewebe im Fußbereich leiden. Durch diese Erkrankungen des Fußes können sich der Abrollvorgang des Fußes und das Gangbild des Patienten verändern und eine Beeinträchtigung der Körperhaltung und Schmerzen verursachen. Zudem ist die Größe der Schwellung und damit die Fußform tages- und tageszeitabhängig unterschiedlich.

Allgemein bekannt sind orthopädische Schuhe, die durch an oder in den Schuh angeordnete Stützelemente eine Unterstützung des Fußes des Patienten bieten sollen. Dazu werden die Schäfte von Schuhen bzw. Stiefeln mit Stützelementen versehen, um dem Fuß Stabilität zu verleihen. Eine Anpassung des Schuhschafts an die durch die Krankheit veränderte Fußform erfolgt durch einstellbare Riemen, Klettverschlüsse oder Schnürungen.

Allgemein sind außerdem Maßschuhe bzw. -stiefel bekannt, die patientenspezifisch in Einzelanfertigung hergestellt werden. Dabei wird von dem erkrankten, funktionsgestörten oder formveränderten Fuß ein Maß- und Formabdruck erstellt und der Schuh nach dieser Maß- und Formvorgabe handwerklich angefertigt. Eine derartige Herstellung von orthopädischen Schuhen ist aufgrund der individualisierten Einzelstückherstellung des Schuhs sehr zeit- und kostenaufwändig. Die individuell hergestellten Schuhe müssen außerdem bei einer Fußformveränderung im Krankheitsverlauf entsprechend angepasst oder gar neu gefertigt werden. Die Neufertigung und die Änderungsschritte führen zu einem hohen Zeit- und Kostenaufwand.

Aus dem Stand der Technik sind orthopädische Schuhe bekannt, die an die Fußformänderung angepasst werden können um dadurch eine Stabilisierung des Fußes des Patienten zu gewährleisten. Derartige orthopädischen Schuhe zur Ausübung von Druck auf den Fußbereich bestehen im Wesentlichen aus dem Schuhschaft, wobei dieser aus einem Oberleder mit Versteifungen im Schaft- und Fußabschnitt und einem flexiblen Futter ausgeführt ist. Außerdem weist der Schuhschaft im Inneren zwei mit Luft aufblasbare Kammern auf, die über eine Pumpeinrichtung mit Luft befüllbar sind. Dadurch dehnen sich die aufblasbaren Kammern aus und üben zur Stabilisierung des Fußes Druck auf den Fußbereich aus. Das Oberleder besteht dabei meist aus einem sehr steifen und schweren Ledermaterial, wodurch das Gewicht des orthopädischen Schuhs sehr hoch ausfällt. Dadurch entsteht ein unangenehmes und beschwerliches Tragegefühl. Außerdem wird durch das sehr steife Ledermaterial das Tragegefühl weiter reduziert und das Abrollverhalten durch die geringe Flexibilität des Schuhschafts bzw. des Schuhs eingeschränkt. Durch eine Schicht des weichen und flexiblen Futters im Inneren des Schuhs kann eine Verbesserung des Tragekomforts des Schuhs erreicht werden. Jedoch muss diese Schicht für einen hohen Tragekomfort besonders dick ausgeführt sein. Durch die Bereitstellung des Platzbedarfs für die dicke innere Schicht aus flexiblem Futter fallen die Außenabmessungen des Schuhs extrem groß und voluminös aus, wodurch ein unnatürliches Tragegefühl und eine sperrige und schwere Handhabung des Schuhs resultieren.

Als Aufgabe der vorliegenden Erfindung wird es daher angesehen, einen orthopädischen Schuh zur Verfügung zu stellen, mit dem eine Stabilisierung des Fußes des Patienten in einem oberen Fußbereich und/oder im Knöchelbereich des Fußes des Patienten besonders gleichmäßig erfolgen kann und eine Anpassung des Schuhs entsprechend einer Fußformveränderung des Patienten möglich ist und gleichzeitig ein hoher Tragekomfort des orthopädischen Schuhs möglich ist.

Diese Aufgabe wird erfindungsgemäß dadurch gelöst, dass das Luftkissen des orthopädischen Schuhs zwei Seitenteile aufweist, wobei beide Seitenteile eine Luftkammer ausbilden, wobei das innere Seitenteil elastisch und das äußere Seitenteil unelastisch ausgeführt ist. Bei einem Aufblasen des Luftkissens wird das elastische innere Seitenteil in Richtung des Fußes ausgelenkt, während das äußere Seitenteil aufgrund der unelastischen Eigenschaft keine Auslenkung nach außen zulässt. Auch durch ein Anlegen eines Innendrucks innerhalb des Luftkissens von einigen Millibar bis einigen Bar erfolgt ausschließlich eine Ausdehnung des Luftkissens in Richtung des Fußes durch eine elastische Ausdehnung des inneren Seitenteils des Luftkissens, wohingegen das unelastische Seitenteil keine elastische Dehnung oder Verformung zulässt. Dadurch legt sich das innere Seitenteil an den Fuß an, wodurch ein Druck zwischen dem Luftkissen und dem Fuß aufgebaut wird. Somit wird der durch das Luftkissen aufgebaute Druck besonders gut in Richtung des Fußes ausgeübt.

Die gewünschten unelastischen Eigenschaften des äußeren Seitenteils werden durch die Verwendung eines unelastischen Materials zur Herstellung des äußeren Seitenteils erreicht. Ein unelastisches Material verhält sich so, dass das Material bei einer Zugbelastung nicht oder lediglich in einem vernachlässigbaren Umfang gedehnt wird. Gleichzeitig kann sich ein unelastisches Material nur in einem geringen Maß an eine Körperform anpassen und anlegen. Vielmehr behält das aus einem unelastischen Material hergestellte äußere Seitenteil unabhängig von der Ausgestaltung des Schuhschafts und unabhängig des an das Luftkissen angelegten Innendrucks die vorgegebene Ausdehnung. Dadurch, dass die Ausdehnung des äußeren Seitenteils sowohl im nicht aufgeblasenen Zustand als auch im aufgeblasenen Zustand des Luftkissens beibehalten wird, ist die Größe und die Position der Kontaktbereiche beziehungsweise der Anlagebereiche zwischen dem äußeren Seitenteil des Luftkissens und dem Schuhschaft in jedem Zustand gleich und damit vorgebbar. Damit kann der Schuhschaft in den vorgegebenen Kontaktbereichen stabil ausgestaltet werden und in den Bereichen, in denen keine Verstärkungselemente notwendig sind, besonders dünn und damit leicht ausgestaltet werden. Somit ist ein insgesamt leichter orthopädischer Schuh mit komfortablen Trageeigenschaften herstellbar. Außerdem kann durch eine derartige Ausgestaltung des Luftkissens das Luftkissen einfach für unterschiedlich gestaltete orthopädische Schuhe verwendet werden, wobei die Gestaltungsmöglichketen der Schuhe dadurch vergrößert ist, dass lediglich geeignete Anlagepunkte für das äußere Seitenteil des Luftkissens an dem Schaft vorgesehen werden müssen.

Die erforderliche Elastizität des inneren Seitenteils kann dagegen gerade durch elastische Materialeigenschaften eines Materials erreicht werden, aus dem das innere Seitenteil gefertigt ist. Ein elastisches Material dehnt sich bei einer Zugbelastung aus und kehrt bei Wegfall der Kraft in den ursprünglichen Zustand zurück. Zudem ist ein elastisches Material mit einem flexiblen Material vergleichbar, das sich besonders gut an eine Körperform anlegen kann.

Es ist aber auch möglich und erfindungsgemäß vorgesehen, dass die erforderliche Elastizität des inneren Seitenteils durch eine Formgebung des inneren Seitenteils bereitgestellt wird. Das innere Seitenteil der Luftkissen kann beispielsweise mit einem Materialüberschuss ausgestaltet sein, wobei sich das innere Seitenteil in einem nicht aufgeblasenen Zustand der Luftkissen in Falten zusammengelegt beziehungsweise zusammengefaltet vorliegt. Durch das Aufblasen der Luftkissen kann sich das innere Seitenteil entsprechend entfalten und sich aufgrund der elastischen Eigenschaften besonders vorteilhaft an die Fußform anpassen und an den Fuß anlegen. Durch die flächige Anlage des Luftkissens an dem Fuß kann eine besonders gleichmäßige Druckverteilung auf den Fuß erfolgen und Druckstellen vermieden werden.

Es ist auch möglich und erfindungsgemäß vorgesehen, dass die gewünschte Elastizität des inneren Seitenteils sowohl durch die elastischen Materialeigenschaften des verwendeten Materials als auch durch die Formgebung des inneren Seitenteils erreicht wird.

Dadurch, dass beide Luftkissen durch die Luftleitung miteinander verbunden sind, werden die beiden Luftkissen zeitgleich aufgeblasen. Dadurch wird der in den Luftkammern der Luftkissen aufgebaute Druck auf beiden Seiten des Fußes besonders gleichmäßig aufgebaut und eine gleichmäßige Stabilisierung des Fußes ermöglicht. Je nach patientenindividueller Fußform können die Luftkissen in ihrer Form und Größe derartig ausgeführt sein, dass sich die Luftkissen über den oberen Fußbereich und/oder über den Knöchelbereich erstrecken.

Um eine Ausdehnung des Luftkissens auf der inneren Seite zuzulassen und eine Ausdehnung des Luftkissens auf der äußeren Seite zu verhindern kann erfindungsgemäß vorgesehen sein, dass die Seitenteile des Luftkissens aus einem Kunststoff-, Gummi- oder Ledermaterial bestehen. Dabei kann das innere Seitenteil beispielsweise aus einem elastischen Kunststoff- oder Gummimaterial bestehen, wodurch das innere Seitenteil besonders leicht durch den in der Luftkammer des Luftkissens aufgebaute Druck ausgedehnt wird und sich an den Fuß anlegt. Außerdem kann sich das innere Seitenteil besonders vorteilhaft an die Fußform anpassen und an den Fuß anlegen. Dabei kann als ein Kunststoffmaterial beispielsweise ein Mikrofasermaterial gewählt werden, das ein besonders angenehmes Tragegefühl erzeugt. Das äußere Seitenteil besteht vorzugsweise aus einem unelastischen Kunststoff oder Ledermaterial, sodass eine Ausdehnung des Luftkissens nach außen verhindert werden kann.

Für einen besonders guten mechanischen Schutz des Fußes, kann eine vorteilhafte Ausgestaltung des orthopädischen Schuhs vorsehen, dass der Schuhschaft eine äußere Lage aufweist, wobei die äußere Lage aus Leder oder Kunststoff besteht. Leder und besonders plastische Kunststoffe weisen eine besonders hohe mechanische Belastbarkeit auf. Außerdem kann dadurch ein stabiler Schuhschaft hergestellt werden, sodass eine besonders gute Stabilisierung des Fußes gewährleistet werden kann. Dadurch, dass die Stabilität des Schuhs zur Stabilisierung des Fußes sowohl durch die Luftkissen als auch durch die äußere Lage des orthopädischen Schuhs erzeugt wird, kann die äußere Lage besonders dünn ausgeführt werden. Dadurch kann ein leichter orthopädischer Schuh mit einem hohen Tragekomfort hergestellt werden. Gleichzeitig wird durch die Luftkissen eine hohe Stabilität gewährleistet, sodass besonders gute Abrolleigenschaften und ein hoher Tragekomfort des Schuhs ermöglicht werden.

Bei einer vorteilhaften Ausgestaltung des erfindungsgemäßen orthopädischen Schuhs ist optional vorgesehen, dass die äußere Lage des Schuhschafts Luftkissenanlageelemente aufweist. Die Luftkissenanlageelemente sind vorteilhafterweise in den Bereichen der äußeren Lage angeordnet, in denen die Luftkissen mit den äußeren Seitenteilen an der äußeren Lage anliegen. Dadurch kann eine Ausdehnung der Luftkissen nach außen verhindert werden.

Es ist aber auch möglich und erfindungsgemäß vorgesehen, dass das äußere Seitenteil des Luftkissens und das Luftkissenanlageelement so geformt sind, dass eine flächige Anlagefläche zwischen dem äußeren Seitenteil des Luftkissens und dem Luftkissenanlageelement entsteht. Dies kann durch eine vorgegebene Formgebung des aus einem unelastischen Material bestehenden Luftkissenanlageelements erreicht werden. Somit kann der durch die Luftkissen erzeugte Druck besonders gut nach innen in Richtung des Fußes übertragen wird, sodass eine besonders gute Stabilisierung des Fußes möglich ist.

Eine vorteilhafte Ausgestaltung der Erfindung sieht vor, dass die Luftkissenanlageelemente aus hartem Leder und/oder Kunststoff bestehen. Dadurch kann ein besonders stabiler Schuhschaft erzeugt werden und eine Ausdehnung des Schuhschafts nach außen verhindert werden. Dadurch, dass das Luftkissenanlageelement und das äußere Seitenteil des Luftkissens aus einem harten Leder und/oder Kunststoff ausgestaltet sind, unterscheiden sich die zwischen dem Luftkissenanlageelement und dem äußeren Seitenteil des Luftkissens ausgebildeten Kontaktbedingungen von den Kontaktbedingungen, die zwischen einem aus einem harten Element und einem weichen Element gebildeten Kontaktpaar ausgebildet sind. Durch eine entsprechende Formgebung des Luftkissenanlageelements kann eine Lage und Positionierung relativ zueinander, beispielsweise durch miteinander korrespondierende und ineinander eingreifende Vertiefungen oder Auswölbungen, vorgegeben sein. Dadurch können die Lage und Positionierung des Luftkissenanlageelement und des äußeren Seitenteils während des Gebrauchs des orthopädischen Schuhs beibehalten werden. Der erfindungsgemäße orthopädische Schuh kann somit besonders stabil ausgestaltet werden.

Um einen stabilen und gleichzeitig leichten orthopädischen Schuh herzustellen, kann es erfindungsgemäß vorgesehen sein, dass die Luftkissenanlageelemente an die Größe der Luftkissen angepasst sind. Somit können die Luftkissenanlageelemente derartig ausgestaltet sein, dass diese eine Ausdehnung des Schuhschafts nach außen verhindert. Durch eine derartige Anpassung der Luftkissenanlageelemente können diese besonders klein ausgeführt werden, sodass das Gewicht der Luftkissenanlageelemente und damit des Schuhs besonders gering ausfällt. Damit wird ein besonders hoher Tragekomfort des orthopädischen Schuhs ermöglicht. Außerdem kann durch die an die Größe der Luftkissen angepassten Luftkissenanlageelementen die Ausdehnung des Luftkissens besonders gezielt und gleichmäßig nach innen in Richtung des Fußes erfolgen. Somit ist eine besonders gute Stabilisierung des Fußes möglich.

Um einen besonders leichten orthopädischer Schuh herzustellen, ist in einer vorteilhaften Ausgestaltung des Erfindungsgedankens vorgesehen, dass das Luftkissenanlageelement so dimensioniert und geformt ist, dass das Luftkissenanlageelement an einer dem Fuß zugewandten Seite des Luftkissenanlageelements ausschließlich mit dem äußeren Seitenteil des Luftkissens in Anlage ist und an einer dem Fuß abgewandten Seite des Luftkissenanlageelements ausschließlich mit der äußeren Lage des Schuhschafts in Anlage ist. Dazu weist das Luftkissenanlageelement vorteilhafterweise kleine Anlageflächen auf beiden Seiten des Luftkissenanlageelements auf, in denen der Kontakt zwischen dem Luftkissenanlageelement und der äußeren Lage des Schuhschafts beziehungsweise dem äußeren Seitenteil des Luftkissens hergestellt ist. Das Luftkissenanlageelement kann in den Anlagebereichen beispielsweise Verstärkungsrippen aufweisen, wohingegen die sich nicht mit der äußeren Lage oder dem Luftkissen in Kontakt befindlichen Bereiche des Luftkissenanlageelements besonders dünn ausgestaltet werden können. Somit ist ein besonders leichter orthopädischer Schuh herstellbar.

Um ein besonders gewichtsoptimiertes Luftkissenanlagenelement herstellen zu können, ist in einer vorteilhaften Ausgestaltung des erfindungsgemäßen orthopädischen Schuhs vorgesehen, dass das Luftkissenanlageelement und das äußere Seitenteil des Luftkissens schalenförmig ausgestaltet sind, sodass bei der Anlage des äußeren Seitenteils des Luftkissens an das Luftkissenanlageelement eine oder mehrere punkt- oder linienförmige Kontaktbereiche entstehen. Erfindungsgemäß sind nur in den punkt- oder linienförmigen Kontaktbereichen Verstärkungselemente, wie beispielsweise Verstärkungsrippen, vorgesehen, wobei die übrigen Bereiche dünnwandig und damit leicht ausgestaltet werden können. Somit ist ein besonders leichter orthopädischer Schuh herstellbar.

Bei einer vorteilhaften Ausgestaltung des erfindungsgemäßen orthopädischen Schuhs ist vorgesehen, dass der Schuhschaft eine innere Lage aufweist, wobei die innere Lage mindestens abschnittsweise ein flexibles Futter aufweist. Die innere Lage kann beispielsweise zwischen dem Luftkissen und dem Fuß angeordnet sein, sodass das flexible Futter am Fuß anliegt. Dabei kann das flexible Futter beispielsweise aus einem weichen Material wie Fell, Kunststofffasern oder einem weichen Leder bestehen. Durch derartige Materialien können besonders angenehme Trageeigenschaften, bei gleichzeitig hoher Atmungsaktivität und guter Geruchshemmung erzeugt werden.

Eine vorteilhafte Umsetzung des Erfindungsgedankens sieht vor, dass die zwei Luftkissen zwischen der äußeren Lage und der inneren Lage des Schuhschafts angeordnet sind. Somit kann der Schuhschaft im Inneren derartig ausgeführt sein, dass der gesamte durch den Schuhschaft umfasste Fuß mit dem flexiblen Futter der inneren Lage in Berührung kommt. Somit ist ein besonders hoher Tragekomfort des orthopädischen Schuhs möglich. Da der durch die Ausdehnung des Luftkissens entstehende Druck über das flexible Futter auf den Fuß übertragen wird, kann eine besonders gleichmäßige Druckverteilung auf den Fuß erfolgen und unangenehme Druckstellen vermieden werden.

Um die Luftleitung und/oder die Luftzuführleitung besonders eng an der äußeren Seite des orthopädischen Schuhs anliegend verlegen zu können, ist es erfindungsgemäß vorgesehen, dass die zwei Luftkissen an den äußeren Seitenteilen jeweils mindestens ein rechtwinkliges Anschlussstück aufweisen, an denen die Luftleitung und/oder die Luftzuführleitung lösbar miteinander verbindbar sind. Dabei weisen die rechtwinkligen Anschlussstücke der Luftkissen nach außerhalb der äußeren Lage des Schuhschafts heraus. Durch eine rechtwinklige Ausgestaltung der Anschlussstücke können die Außenabmessungen des Schuhs besonders klein gehalten werden. Außerdem kann ein Verhaken und ein Hängenbleiben beim Tragen des orthopädischen Schuhs vermieden werden und ein damit verbundenes Verletzungsrisiko minimiert werden. Dadurch, dass das mindestens eine Anschlussstück an dem äußeren Seitenteil der Luftkissen angebracht ist, kommt dieses nach dem Aufblasen des Luftkissens nicht in Kontakt mit dem Fuß des Patienten. Somit ist ein besonders hoher Tragekomfort des orthopädischen Schuhs im aufgeblasenen Zustand des Luftkissens gewährleistet. Durch die lösbare Verbindung der Luftleitung und/oder der Luftzuführleitung mit den Anschlussstücken können die Luftleitung und/oder die Luftzuführleitung im Schadensfall besonders einfach, schnell und kostengünstig ausgetauscht werden.

Eine vorteilhafte Ausgestaltung der Erfindung sieht vor, dass die Luftleitung und/oder die Luftzuführleitung aus einem Kunststoff-, Gummi- oder Silikonmaterial bestehen/besteht, da diese Materialien besonders widerstandsfähig gegen äußere Umwelteinflüsse, wie Staub, Feuchtigkeit und Schmutz sind. Zudem verfügen diese Materialien über eine hohe Flexibilität, sodass die Luftleitung und die Luftzuführleitung besonders einfach auf der Außenfläche des Schuhschafts verlegt werden können. In einer erfindungsgemäßen Ausgestaltung des orthopädischen Schuhs kann außerdem vorgesehen sein, dass die Luftleitung und die Luftzuführleitung durch einen Klettverschluss an der Außenfläche des Schuhs festgelegt werden können. Ein zusätzlicher Schutz gegen mechanische Einflüsse kann beispielsweise durch einen Schutzdeckel, der über die Luftleitung und Luftzuführleitung gelegt wird, erreicht werden.

Bei einer vorteilhaften Ausgestaltung des erfindungsgemäßen orthopädischen Schuhs ist vorgesehen, dass die Pumpeinrichtung als Handpumpe oder elektrische Pumpe ausgeführt ist. Die Pumpeinrichtung wird über die Luftzuführleitung an das Luftkissen angeschlossen. Dadurch, dass die Luftzuführleitung über ein Rückschlagventil verfügt, kann die Pumpeinrichtung nach dem Befüllen des Luftkissens wieder von der Luftzuführleitung getrennt werden, ohne dass die eingepumpte Luft wieder entweicht. Ein Aufpumpen des Luftkissens kann durch eine Handpumpe oder durch eine elektrische Pumpe besonders einfach und schnell erfolgen.

Da die Patienten aufgrund ihrer Erkrankung häufig nichts mehr am Fuß spüren und somit kein Empfinden für einen äußeren Druck empfinden können, ist in einer vorteilhaften Umsetzung des Erfindungsgedankens vorgesehen, dass die Luftzuführleitung oder die Pumpeinrichtung ein Manometer zur Druckanzeige aufweist. Dadurch ist es für die Patienten besonders einfach, den patientenindividuellen Druck innerhalb der Luftkissen einstellen zu können. Außerdem kann die Luftzuführleitung oder die Pumpeinrichtung mit einem Sicherheitsventil ausgestattet sein, sodass ein bestimmter eingestellter Maximaldruck nicht überschritten werden kann. Dadurch können Verletzungen durch ein zu starkes Aufpumpen der Luftkissen verhindert werden.

Zum Ablassen der mittels der Pumpeinrichtung in die Luftkissen eingepumpte Luft aus den Luftkissen kann es erfindungsgemäß vorgesehen sein, dass die Pumpeinrichtung ein Entlüftungsventil aufweist. Dadurch, dass das Entlüftungsventil nicht an dem orthopädischen Schuh, sondern an der abnehmbaren Pumpeinrichtung angebracht ist, kann der orthopädische Schuh besonders klein ausgeführt sein. Nachfolgend werden einige Ausführungsbeispiele des orthopädischen Schuhs näher erläutert, die in den Zeichnungen dargestellt sind. Es zeigen:
Fig. 1 eine schematische Darstellung des orthopädischen Schuhs in einer Seitenansicht und
Fig. 2 eine schematische Darstellung einer Schnittansicht des orthopädischen Schuhs in der in Fig 1 eingezeichneten Schnittebene II-II.

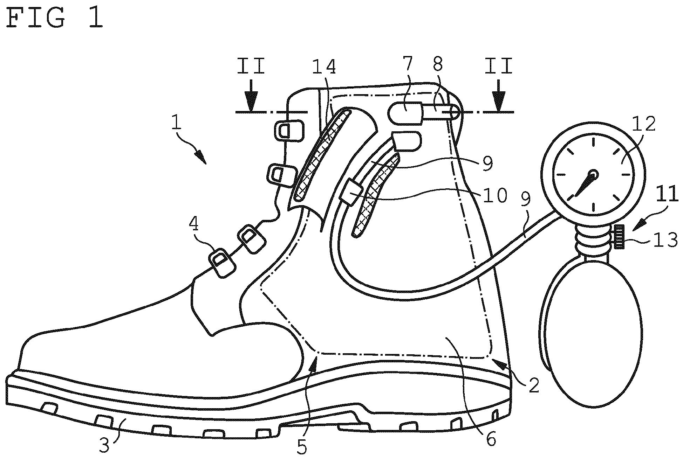

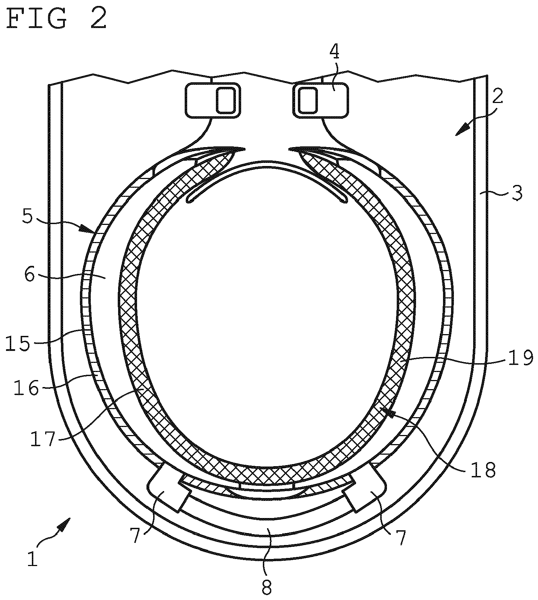

In den Darstellungen der Figuren 1 und 2 sind jeweils einzelne mehrere gleichartige Elemente exemplarisch mit einem Bezugszeichen gekennzeichnet.

In Figur 1 ist ein erfindungsgemäßer orthopädischer Schuh 1, der stiefelartig ausgeführt ist, dargestellt. Der orthopädische Schuh 1 weist einen Schuhschaft 2 auf, der sich entlang der Seiten eines, in Figur 1 nicht dargestellten, Fußes erstreckt und mit einer Sohle 3 und Verschnürungselementen 4 verbunden ist. Der Schuhschaft 2 weist eine äußere Lage 5 auf, die beispielsweise aus einem Ledermaterial besteht. Auf der dem Fuß zugewandten Seite der äußeren Lage 5 des Schuhschafts 2 sind zwei Luftkissen 6 vorgesehen, wobei das eine Luftkissen 6 mit der in Figur 1 dargestellten Strich-Punkt-Linie angedeutet ist. In der dargestellten erfindungsgemäßen Ausführung des orthopädischen Schuhs 1 weisen beide Luftkissen 6 ein rechtwinkliges Anschlussstück 7 auf, die vom Luftkissen 6 nach außerhalb der äußeren Lage 5 des Schuhschafts 2 herausreichen. Beide Luftkissen 6 sind über eine flexible Luftleitung 8 miteinander verbunden, die mit ihren beiden Enden auf die Anschlussstücke 7 der Luftkissen 6 lösbar aufgesteckt sind. Das eine Luftkissen 6 weist außerdem ein weiteres Anschlussstück 7 auf, an das eine flexible Luftzuführleitung 9 angeschlossen ist. Die Luftzuführleitung 9 weist ein Rückschlagventil 10 auf, an das über eine weitere Luftzuführleitung 9 eine Pumpeinrichtung 11 angeschlossen werden kann. Die Pumpvorrichtung 11 ist in diesem Beispiel als Handpumpe ausgeführt. Die Pumpvorrichtung 11 weist ein Manometer 12 auf, durch das der in die Luftkissen 6 eingebrachte Druck kontrolliert werden kann. Außerdem weist die Pumpvorrichtung 11 ein Entlüftungsventil 13 auf, mit dem der Druck der Luftkissen 6 wieder abgelassen werden kann. Die Luftzuführleitung 9 und das Rückschlagventil 10 können durch einen Klettverschluss 14 an dem Schuhschaft 2 festgelegt werden.

In Figur 2 ist eine schematische Darstellung einer Schnittansicht des orthopädischen Schuhs 1 in der in Figur 1 eingezeichneten Schnittebene II-II gezeigt, um den Aufbau des orthopädischen Schuhs 1 darzustellen. Dabei besteht der orthopädische Schuh 1 aus der äußeren Lage 5 des Schuhschafts 2 mit Luftkissenanlageelementen 15, an denen ein äußeres Seitenteil 16 des Luftkissens 6 anliegen. Ein inneres Seitenteil 17 des Luftkissens 6 liegt an einer inneren Lage 18 des Schuhschafts 2 an. Dabei besteht die innere Lage 18 des Schuhschafts 2 aus einem weichen flexiblen Futter 19.

### BEZUGSZEICHENLISTE

- 1.: Orthopädischer Schuh
- 2.: Schuhschaft
- 3.: Sohle
- 4.: Verschnürungselement
- 5.: Äußere Lage
- 6.: Luftkissen
- 7.: Anschlussstück
- 8.: Luftleitung
- 9.: Luftzuführleitung
- 10.: Rückschlagventil
- 11.: Pumpeinrichtung
- 12.: Manometer
- 13.: Entlüftungsventil
- 14.: Klettverschluss
- 15.: Luftkissenanlageelement
- 16.: Äußeres Seitenteil
- 17.: Inneres Seitenteil
- 18.: Innere Lage
- 19.: Flexibles Futter

## Patentansprüche

1. Orthopädischer Schuh (1) zur Stabilisierung eines Fußes in einem oberen Fußbereich und/oder im Knöchelbereich eines Trägers, wobei der orthopädische Schuh (1) einen Schuhschaft (2) aufweist, wobei sich der Schuhschaft (2) mindestens entlang der Seiten des Fußes und über den oberen Fußbereich erstreckt, wobei der Schuhschaft (2) mindestens zwei Luftkissen (6) aufweist, wobei das eine Luftkissen medial, das heißt mittig zur Körperlinie und das andere Luftkissen lateral, das heißt seitlich zur Körperlinie angeordnet ist, wobei beide Luftkissen (6) durch eine Luftleitung (8) miteinander verbindbar sind, wobei mindestens ein Luftkissen (6) eine Luftzuführleitung (9) und ein Rückschlagventil (10) zum Verbinden mit einer Pumpeinrichtung (11) aufweist, **dadurch gekennzeichnet, dass** die Luftkissen (6) jeweils zwei Seitenteile (16, 17) aufweisen, wobei beide Seitenteile (16, 17) eine Luftkammer ausbilden, wobei das innere Seitenteil (16) elastisch und das äußere Seitenteil (17) unelastisch ausgeführt ist.

2. Orthopädischer Schuh (1) nach Anspruch 1, **dadurch gekennzeichnet, dass** die Seitenteile (16, 17) der Luftkissen (6) aus einem Kunststoff-, Gummi- oder Ledermaterial bestehen.

3. Orthopädischer Schuh (1) nach Anspruch 1 oder 2, **dadurch gekennzeichnet, dass** der Schuhschaft (2) eine äußere Lage (5) aufweist, wobei die äußere Lage (5) aus Leder oder Kunststoff besteht.

4. Orthopädischer Schuh (1) nach einem der voranstehenden Ansprüche, **dadurch gekennzeichnet, dass** die äußere Lage (5) des Schuhschafts (2) Luftkissenanlageelemente (15) aufweist.

5. Orthopädischer Schuh (1) nach einem der voranstehenden Ansprüche, **dadurch gekennzeichnet, dass** die Luftkissenanlageelemente (15) aus hartem Leder und/oder Kunststoff bestehen.

6. Orthopädischer Schuh (1) nach einem der voranstehenden Ansprüche, **dadurch gekennzeichnet, dass** die Luftkissenanlageelemente (15) an die Größe der Luftkissen (6) angepasst sind.

7. Orthopädischer Schuh (1) nach einem der voranstehenden Ansprüche, **dadurch gekennzeichnet, dass** das Luftkissenanlageelement (15) so dimensioniert und geformt ist, dass das Luftkissenanlageelement (15) an einer dem Fuß zugewandten Seite des Luftkissenanlageelements (15) ausschließlich mit dem äußeren Seitenteil (17) des Luftkissens (6) in Anlage ist und an einer dem Fuß abgewandten Seite des Luftkissenanlageelements (15) ausschließlich mit der äußeren Lage (5) des Schuhschafts (2) in Anlage ist.

8. Orthopädischer Schuh (1) nach einem der voranstehenden Ansprüche, **dadurch gekennzeichnet, dass** das Luftkissenanlageelement (15) und das äußere Seitenteil (17) des Luftkissens (6) schalenförmig ausgestaltet sind, sodass bei der Anlage des äußeren Seitenteils (17) des Luftkissens (6) an das Luftkissenanlageelement (15) eine oder mehrere punkt- oder linienförmige Kontaktbereiche entstehen.

9. Orthopädischer Schuh (1) nach einem der voranstehenden Ansprüche, **dadurch gekennzeichnet, dass** der Schuhschaft (2) eine innere Lage (18) aufweist, wobei die innere Lage (18) mindestens abschnittsweise ein flexibles Futter (19) aufweist.

10. Orthopädischer Schuh (1) nach einem der voranstehenden Ansprüche, **dadurch gekennzeichnet, dass** die zwei Luftkissen (6) zwischen der äußeren Lage (5) und der inneren Lage (18) des Schuhschafts (2) angeordnet sind.

11. Orthopädischer Schuh (1) nach einem der voranstehenden Ansprüche, **dadurch gekennzeichnet, dass** die zwei Luftkissen (6) an den äußeren Seitenteilen (17) jeweils mindestens ein rechtwinkliges Anschlussstück (7) aufweisen, an denen die Luftleitung (8) und/oder die Luftzuführleitung (9) lösbar miteinander verbindbar sind.

12. Orthopädischer Schuh (1) nach einem der voranstehenden Ansprüche, **dadurch gekennzeichnet, dass** die Luftleitung (8) und/oder die Luftzuführleitung (9) aus einem Kunststoff-, Gummi- oder Silikonmaterial bestehen/besteht.

13. Orthopädischer Schuh (1) nach einem der voranstehenden Ansprüche, **dadurch gekennzeichnet, dass** die Pumpeinrichtung (11) als Handpumpe oder elektrische Pumpe ausgeführt ist.

14. Orthopädischer Schuh (1) nach einem der voranstehenden Ansprüche, **dadurch gekennzeichnet, dass** die Luftzuführleitung (9) oder die Pumpeinrichtung (11) ein Manometer (12) zur Druckanzeige aufweist.

15. Orthopädischer Schuh (1) nach einem der voranstehenden Ansprüche, **dadurch gekennzeichnet, dass** die Pumpeinrichtung (11) ein Entlüftungsventil (13) aufweist.
